(19) Europäisches Patentamt
European Patent Office
Office européen des brevets

(11) Veröffentlichungsnummer: **0 027 554**
**B1**

(12) **EUROPÄISCHE PATENTSCHRIFT**

(45) Veröffentlichungstag der Patentschrift: **02.11.83**

(51) Int. Cl.³: **C 07 C 17/34**, C 07 C 1/30, C 07 C 21/06, B 01 J 19/12

(21) Anmeldenummer: **80105649.0**

(22) Anmeldetag: **19.09.80**

(54) **Verfahren zur Herstellung von olefinischen Doppelbindungen durch Halogenwasserstoffabspaltung.**

(30) Priorität: **21.09.79 DE 2938353**
**07.03.80 DE 3008848**

(43) Veröffentlichungstag der Anmeldung: **29.04.81 Patentblatt 81/17**

(45) Bekanntmachung des Hinweises auf die Patenterteilung: **02.11.83 Patentblatt 83/44**

(84) Benannte Vertragsstaaten: **BE CH FR GB IT LI NL SE**

(56) Entgegenhaltungen:
**US - A - 4 165 269**

**JOURNAL OF THE AMERICAN CHEMICAL SOCIETY Band 100, Nr. 7, 1978 Columbus R.N. ZITTNER et al. "Enthalpy Limit in the Laser-Induced Reaction of** ***CH3CF2Cl" Seiten 2265 bis 2266***
**Chemical Abstracts, Band 90, Nr. 1, 1. Januar 1979 Columbus, Ohio, USA F.M. LUSSIER et al. "Pressure and intensity dependence of multiphoton energy deposition and reaction yield in vinyl chloride" Seite 528, Spalte 2, Abstract Nr. 5569k**

(73) Patentinhaber: **Max-Planck-Gesellschaft zur Förderung der Wissenschaften e.V., Bunsenstrasse 10, D-3400 Göttingen (DE)**

(72) Erfinder: **Wolfrum, Jürgen, Dr., Südring 2, D-3405 Rosdorf (DE)**
Erfinder: **Kneba, Michael, Dr., Weserstrasse 21, D-3400 Göttingen (DE)**
Erfinder: **Clough, Peter N., Dr., 13, Ivanhoe Avenue, Belfast 8, N. Ireland (GB)**
Erfinder: **Schneider, Matthias, Rosdorfer Weg 32, N-3400 Göttingen (DE)**

(74) Vertreter: **Weickmann, Heinrich, Dipl.-Ing. et al, Patentanwälte Dipl.-Ing. H. Weickmann Dipl.-Phys.Dr. K. Fincke Dipl.-Ing. F.A. Weickmann Dipl.-Chem. B. Huber Dr.-Ing. H. Liska Möhlstrasse 22, D-8000 München 86 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 027 554 B1**

ACTORUM AG

# Verfahren zur Herstellung von olefinischen Doppelbindungen durch Halogenwasserstoffabspaltung.

Die Erfindung betrifft ein Verfahren zur Herstellung von gegebenenfalls halogenierten Verbindungen mit wenigstens einer olefinischen Doppelbindung durch Abspaltung von Halogenwasserstoff aus der entsprechenden, Halogen und Wasserstoff enthaltenden gesättigten Verbindung durch Radikalkettenreaktion.

Es ist bekannt, dass viele chemische Reaktionen sowohl in der Gasphase als auch in der flüssigen Phase in Form einer sogenannten Kettenreaktion ablaufen. Eine Reaktionskette erhält man, wenn als Folge eines einzigen Aktivierungsschritts eine grosse Zahl von Molekülen umgesetzt wird.

Schreibt man stabile Ausgangsprodukte als $A_1$, $A_2$..., Endprodukte als $B_1$, $B_2$... und aktive Zwischenprodukte als $X_1$, $X_2$..., so gilt das allgemeine Schema:

| | |
|---|---|
| a) $A_1 \rightarrow X_1 + B_1$ | Kettenstart |
| b) $X_1 + A_2 \rightarrow B_2 + X_2$ | Kettenfortführung |
| c) $X_2 + A_3 \rightarrow B_3 + nX_1$ | Kettenfortführung (für $n > 1$ mit Kettenverzweigung). |

Da sich die Zyklen b) und c) im Prinzip beliebig oft wiederholen können, läuft die Reaktionsfolge ab, wenn ein aktives Teilchen $X_1$ in die Reaktion eingetreten ist, bis das Teilchen $X_1$ schliesslich nach einer Reaktion verbraucht und nicht wieder regeneriert wird. Die Zahl der pro eingeführtes Teilchen $X_1$ ablaufenden Schritte b) und c) bezeichnet man als Kettenlänge. Diese kann mehrere Zehnerpotenzen betragen.

Bei den vorwiegend angewandten technischen Verfahren, in denen Kettenreaktionen eine Rolle spielen, werden die aktiven Teilchen $X_1$ überwiegend thermisch, d.h. durch unspezifische Aufheizung (sogenannte «Pyrolyse») in den Reaktionsablauf eingeführt. Hierzu sind im allgemeinen relativ hohe Temperaturen erforderlich. Die zur Pyrolyse notwendigen hohen Temperaturen erfordern einen grossen Energieaufwand und führen zudem häufig zu unerwünschten Nebenreaktionen. Der Hauptenergieanteil wird bei den thermischen Verfahren zur Spaltung der Reaktanden und damit zur Freisetzung der aktiven Teilchen, d.h. für die ketteneinleitende Reaktion, aufgebraucht. Die Reaktionen, welche die Kette fortführen (b) und (c) und entsprechend der Kettenlänge den weitaus grösseren Anteil an dem gesamten Reaktionsumsatz ausmachen, sind im allgemeinen bereits bei erheblich niedrigeren Temperaturen als den zur Pyrolyse notwendigen hinreichend schnell. Hieraus ist ersichtlich, dass eine effiziente Methode zur Bereitstellung der aktiven Teilchen $X_1$ bei vergleichsweise niedrigen Temperaturen eine Reihe erheblicher Vorteile mit sich bringen würde.

Es ist bekannt, derartige aktive Teilchen z.B. durch Zuführung von Zusätzen, die relativ leicht aktive Teilchen $X_1$ thermisch freisetzen, zu erzeugen. Als Beispiel dafür sei z.B. der Zusatz von $Cl_2$ (allg. $X_2$) genannt, wenn $X_1$ ein freies Chloratom ist. Solche Zusätze beinhalten jedoch häufig Nachteile, wie zusätzliche Kosten und z.B. Korrosionsprobleme. Andere bekannte Verfahren erzeugen die Radikale $X_1$ durch Photolyse mit herkömmlichen Lichtquellen, z.B. mit Quecksilber-Hochdrucklampen. Diese Lichtquellen haben jedoch nur eine relativ geringe Ausnutzung der zugeführten Energie in für den gewünschten Prozess nutzbarer Photonenenergie. Ausserdem liefern diese Lichtquellen nur eine relativ geringe Photonendichte in einem engen Spektralbereich.

Die vorstehend erläuterten Schwierigkeiten treten besonders auf bei der Herstellung von Verbindungen, welche eine oder mehrere olefinische Doppelbindungen aufweisen durch Halogenwasserstoffabspaltung aus der entsprechenden, Halogen und Wasserstoff enthaltende gesättigten Verbindung. Die erhaltene olefinisch ungesättigte Verbindung kann dabei noch ein oder mehrere weitere Halogenatome enthalten. Ein typisches Beispiel hierfür ist die Herstellung von monomerem Vinylchlorid (VCM) aus Dichloräthan.

Bei diesem grosstechnisch durchgeführten Verfahren wird 1,2-Dichloräthan in einem mehrere hundert Meter langen Pyrolyseofen bei etwa 500 °C zu Vinylchlorid und Chlorwasserstoff gespalten. Der Hauptanteil der Reaktion läuft dabei über die einfache homogene Abspaltung von Chlorwasserstoff aus Dichloräthan. Wegen der dabei erforderlichen hohen Reaktionstemperatur sind nicht nur grosse Wärmemengen für die Aufheizung erforderlich, sondern es entsteht auch eine Reihe unerwünschter Nebenprodukte, die bei dem weiteren Ablauf des Prozesses erheblich stören und deshalb mit aufwendigen Verfahren abgetrennt werden müssen.

Unter den oben angegebenen Bedingungen beträgt dabei die Umwandlung etwa 50 bis 60% und die Selektivität 96 bis 99% (vgl. Hydrocarbon Processing, März 1979, Seiten 75–88).

Der Erfindung liegt daher die Aufgabe zugrunde, ein Verfahren der eingangs erwähnten Art zu schaffen, bei welchem die zur Erzielung der Radikalkettenreaktion erforderliche Energie herabgesetzt wird, Umwandlung und Selektivität erhöht sind, praktisch keine störenden Nebenprodukte gebildet werden, zur Inaktivierung bzw. Vergiftung neigende Katalysatoren nicht erforderlich sind und die Reaktionsdauer verkürzt wird.

Gelöst werden diese Aufgaben erfindungsgemäss durch ein Verfahren zur Herstellung von gegebenenfalls halogenierten Verbindungen mit wenigstens einer olefinischen Doppelbindung durch Abspaltung von Halogenwasserstoff aus der entsprechenden, Halogen und Wasserstoff enthaltenden gesättigten Verbindung, welches dadurch gekennzeichnet ist, dass man die gasförmige gesättigte Verbindung in einem Reaktionsraum mit gepulstem kohärentem Licht oder/und inkohärentem Licht bestrahlt und die Wellenlänge des eingestrahlten Lichts und die Druck- und Temperaturbedingungen im Reaktionsraum so wählt, dass

sich ein Einfangquerschnitt von $10^{-15}$ bis $10^{-25}$ $cm^2$/Molekül ergibt.

Gemäss einer ersten Ausführungsform des erfindungsgemässen Verfahrens ist die Verwendung von kohärentem Licht (Laser) wesentlich. Hierdurch wird es möglich, eine sehr hohe Photonendichte und hohe Leistung zu erzielen und monochromatisches Licht sehr scharfer Wellenlänge zu erzeugen. Zusätzlich besteht die Möglichkeit, sehr kurze Lichtimpulse hoher Leistung zu erhalten. Diese vorstehend aufgeführten Eigenschaften der kohärenten Laserlichtquellen ermöglichen eine selektive und effektive Erzeugung von aktiven Teilchen $X_1$ (wie oben beschrieben) unter Bedingungen, bei denen dies mit herkömmlichen Lichtquellen nicht oder nur in sehr geringem Umfang möglich ist. Die hohe Monochromasie der Laserstrahlung erlaubt eine sehr selektive Anregung der die aktiven Teilchen $X_1$ freisetzenden Stoffe durch die Auswahl geeigneter Energieübergänge (Absorptionslinien). Die hohe Photonendichte der Laserstrahlung gestattet es, zusätzlich durch Laserphotolyse in kurzer Zeit eine sehr hohe Konzentration reagierender aktiver Teilchen $X_1$ für die gewünschte Kettenreaktion in einem relativ grossen Volumen in einem weiten Temperatur- und Druckbereich homogen zu erzeugen. Dadurch ist ein rascher Reaktionsumsatz möglich. Die hohe Photonendichte von Laserlichtquellen lässt nun nicht nur die auch bei herkömmlichen Lichtquellen bekannte Anregung durch Einzelphotonenabsorption zu; es sind auch Zwei- und Mehrfachphotonenabsorptionen möglich. Durch Zwei- und Mehrfachphotonenabsorption lassen sich Moleküle in höhere Energiezustände anregen als dies bei Einfachphotonenabsorption von Licht der gleichen Wellenlänge geschieht. Die höhere Anregung bewirkt, dass die angeregten Moleküle schneller zerfallen.

Ein zweites wesentliches Merkmal des erfindungsgemässen Verfahrens besteht darin, dass die Wellenlänge des eingestrahlten Laserlichts und die Druck- und Temperaturbedingungen im Reaktionsraum so gewählt werden, dass man den oben bereits erwähnten Einfangquerschnitt von $10^{-15}$ bis $10^{-25}$ $cm^2$/Molekül erhält. Anders ausgedrückt wird erfindungsgemäss bei Wellenlängen gearbeitet, die von den jeweiligen Molekülen nur wenig absorbiert werden. Überraschenderweise wurde nämlich gefunden, dass ein relativ kleiner Absorptionsquerschnitt wesentlich günstiger ist, obwohl man bisher stets einen möglichst grossen Absorptionsquerschnitt angestrebt hat in der Annahme, damit die beste Energieausbeute erzielen zu können.

Allgemeiner ausgedrückt muss erfindungsgemäss eine solche Wellenlänge für das Laserlicht verwendet werden, die es ermöglicht, den gesamten Reaktionsraum praktisch gleichmässig zu durchleuchten. Auf diese Weise wird verhindert, dass das eingestrahlte Licht infolge eines hohen Absorptionsquerschnitts schon nach Durchlaufen nur eines Teils des Reaktionsraumes vollständig absorbiert ist. Letzteres ist jedoch der Fall, wenn, wie bisher angestrebt wurde, die Wellenlänge der eingestrahlten Strahlung möglichst gut der Absorptionswellenlänge der umzusetzenden Verbindung entspricht. Erfindungsgemäss wird von diesen Bedingungen erheblich abgewichen und nur eine relativ geringfügige Absorption angestrebt.

Die Einführung eines aktiven Teilchens $X_1$ in ein solches Reaktionssystem wird also erfindungsgemäss dadurch erzielt, dass für die jeweils interessierende Reaktion geeignete Moleküle durch Laserstrahlung derart angeregt werden, dass sie unter Bildung von aktiven Teilchen $X_1$ zerfallen:

$$A_1 + nh\nu_{Laser} \rightarrow A_1^{\neq} \text{ (angeregt)} \rightarrow X_1 + B_1 \; (n \geq 1).$$

«n» bedeutet dabei die Zahl der pro Molekül der gesättigten, Halogen und Wasserstoff enthaltenden Verbindung $A_1$ absorbierten Laserquanten. Dieser Vorgang wird als Laserphotolyse bezeichnet. Als Laserlichtquelle kommt dabei eines der zahlreichen bekannten Lasersysteme sowohl im ultravioletten, sichtbaren als auch im infraroten Spektralbereich zur Anwendung. Bevorzugt wird ein Excimerenlaser.

Die Wellenlänge der dabei zweckmässigerweise eingesetzten Laserstrahlung hängt in der oben schon erläuterten Weise ab von den spektroskopischen und photochemischen Eigenschaften des Moleküls $A_1$ sowie von dem Druck und der Temperatur. Sie kann sowohl im Vakuum-Ultraviolett-Bereich, im sichtbaren als auch im infraroten Spektralbereich liegen.

Gemäss einer speziellen Ausführungsform des erfindungsgemässen Verfahrens kann man den Einfangquerschnitt auch durch Zusatz kleiner Mengen an höher halogenierten Verbindungen, Halogenwasserstoff oder Halogen regeln.

Mit dem erfindungsgemässen Verfahren der Laserphotokatalyse gelingt eine schnelle quantitative Abspaltung von Chlorwasserstoff aus 1,2-Dichloräthan bereits bei erheblich niedrigeren Temperaturen als in einem rein thermischen Verfahren. Die Umsetzung verläuft zusätzlich weitgehend frei von störenden Nebenreaktionen, die zu anderen Produkten als Vinylchlorid und Chlorwasserstoff führen.

Die als Beispiel aufgeführte Reaktion läuft unter den Bedingungen einer Laserphotokatalyse etwa nach folgendem Schema ab: Zunächst erfolgt die Einleitung der Reaktionskette durch Laserphotolyse von 1,2-Dichloräthan ($C_2H_4Cl_2$)

$$1)\; C_2H_4Cl_2 + nh\nu_{Laser} \xrightarrow{\text{Anregung}} (C_2H_4Cl_2)$$

$$\text{angeregt} \xrightarrow{\text{Zerfall}} \text{z.B. } C_2H_4Cl + Cl.$$

Die durch die Laserphotolyse entstehenden freien Cl-Atome entsprechen den aktiven Teilchen $X_1$ im vorstehend angegebenen allgemeinen Reaktionsschema und können nun in einer Kettenreaktion Dichloräthan unter Bildung von Vinylchlorid umsetzen:

$$2)\qquad C_2H_4Cl_2 + Cl \rightarrow C_2H_3Cl_2\cdot + HCl,$$

wobei das zunächst entstehende Zwischenprodukt

$C_2H_3Cl_2\cdot$ nach

3) $C_2H_3Cl_2\cdot \rightarrow C_2H_3Cl + Cl$

zu Vinylchlorid zerfällt, wodurch das in Schritt 2) verbrauchte freie Cl-Atom gleichzeitg wieder in die Reaktionskette zurückgeführt wird.

Vorzugsweise wird eine Strahlung einer Impulsdauer bis herab zu $10^{-15}$ Sekunden und einer Energie von 0,01 bis 100 Joule verwendet. Dies gilt auch für analoge, unter Halogenwasserstoffabspaltung verlaufende Reaktionen mit anderen gesättigten Ausgangsprodukten. Im Rahmen der Erfindung werden dabei unter Halogen Chlor, Brom, Jod und Fluor verstanden.

Bei der VCM-Herstellung aus Dichloräthan beträgt die Temperatur zweckmässig 200 bis 550 °C, vorzugsweise 230 bis 320 °C. Der Druck liegt zweckmässig zwischen 50 und 3000 kPa, vorzugsweise 0,1 bis 1 MPa. Jedoch kann auch ausserhalb der angegebenen Bereiche gearbeitet werden. So lässt sich die Umsetzung bei Temperaturen zwischen 10 und 700 °C durchführen. Wie bereits erwähnt, werden vorzugsweise Excimerenlaser verwendet, wobei nicht nur solche in Betracht kommen, die im UV-Bereich liegen, sondern auch solche im sichtbaren oder infraroten Spektralbereich. Zur Regelung des Einfangquerschnitts können beispielsweise $Cl_2$, HCl, $CFCl_3$, $CF_2Cl_2$ zugesetzt werden. Diese Zusätze erhöhen den Einfangquerschnitt und spalten freie Cl-Atome ab, welche als Radikalketteninitiatoren wirksam werden. Sie ermöglichen es damit, die Zahl der Reaktionsketten und damit auch die Länge der Reaktionsketten zu variieren. Strebt man z. B. eine Kettenlänge von $10^4$ an, d.h. $10^4$-Abspaltungsvorgänge pro initiierendem Radikal, so würde das Verhältnis Dichloräthan zu Zusatzstoff höchstens $10^{-4}$ Mol pro Mol Dichloräthan betragen. Als Ausgangsstoff eignet sich besonders 1,2-Dichloräthan, es kann aber auch 1,1-Dichloräthan verwendet werden.

Für diese bevorzugte Ausführungsform der Erfindung geeignete Laserstrahlungsquellen sind beispielsweise Nd:YAG ($\lambda$ = 265 nm); KrF ($\lambda$ = 249 nm); KrCl ($\lambda$ = 225 nm); ArF ($\lambda$ = 193 nm). Andere geeignete Laser sind z.B. ein Farbstofflaser im sichtbaren Spektralbereich oder ein $CO_2$-Laser im Infrarotbereich.

Weiter wurde gefunden, dass bei einem derartigen Verfahren auch sehr gute Resultate erreicht werden, wenn man die bekannten thermischen Verfahren (Pyrolyse) mit dem Verfahren der Photokatalyse kombiniert. Dabei wird ausgenutzt, dass die chemische Umsetzung bei den beiden Verfahren auf verschiedenen und unabhängigen Wegen abläuft. Insbesondere ist es dadurch auch möglich, bereits vorhandene Anlagen für das thermische Verfahren in wirtschaftlich wenig aufwendiger Weise mit dem Verfahren der Photokatalyse zu kombinieren, wodurch aufwendige technische Neukonstruktionen vermieden werden können.

Gemäss einer zweiten Ausführungsform der Erfindung wird daher die gesättigte Verbindung in einem Reaktionsraum thermisch behandelt und mit kohärentem und/oder inkohärentem Licht bestrahlt.

Die Reaktionsbedingungen für diese kombinierte Ausführungsform der Erfindung (z.B. Druck, Temperatur, Strömungsgeschwindigkeit der Reaktanden, gegebenenfalls Zusätze) können dabei entsprechend den Reaktionsbedingungen der bekannten thermischen Verfahren und den oben für die erste Ausführungsform beschriebenen Bedingungen gewählt werden.

Vorzugsweise arbeitet man bei der kombinierten Ausführungsform bei Temperaturen zwischen 200 und 600 °C, insbesondere zwischen 450 und 550 °C; man kann unter Normaldruck oder auch reduziertem Druck arbeiten, vorzugsweise aber unter erhöhtem Druck. Als besonders vorteilhaft hat sich dabei ein Druck von 10 bis 30 at erwiesen, weil dadurch eine verbesserte Trennung der Reaktionsprodukte, insbesondere eine verbesserte Abtrennung des Halogenwasserstoffs, erreicht werden kann. Die Verwendung kleiner Einfangquerschnitte wird dabei durch die Kombination mit dem thermischen Verfahren noch weiter begünstigt.

Als inkohärente Lichtquelle verwendet man vorzugsweise Metalldampfmitteldrucklampen oder auch Metalldampfniederdrucklampen, insbesondere mit einem Quantenflussbereich von 0,01 bis 10 W/cm², vor allem von 0,1 bis 2 W/cm². Als zweckmässig haben sich dabei Quecksilber-, Thallium- und/oder Eisenmetalldampflampen erwiesen. Die Wellenlängen liegen dabei vorzugsweise zwischen 250 und 350 nm. Im Vergleich zu Laseranordnungen erfordern solche Lampen wesentlich geringere Investitions- und Betriebskosten.

Die thermische Behandlung und die Bestrahlung können gleichzeitig oder in hintereinanderfolgenden Stufen erfolgen. Die Reaktanden können im Reaktionsraum stehen. Zweckmässigerweise wird aber, vor allem wenn man Mitteldruck- oder Niederdrucklampen verwendet, so gearbeitet, dass die Reaktanden den Reaktionsraum durchströmen.

Es hat sich nämlich gezeigt, dass bei der Verwendung von Mitteldruck- oder Niederdrucklampen die Fenster durch Nebenprodukte rasch bedeckt werden. Dieses Problem tritt bei der Verwendung von Laserstrahlen nicht störend in Erscheinung, da hier durch die höhere Energie die auf den Festern abgelagerten Partikelchen wieder weggerissen werden. Es empfiehlt sich deshalb bei inkohärenten Lichtquellen eine Arbeitsweise, bei welcher mit relativ hoher Strömungsgeschwindigkeit am Bestrahlungsfenster vorbei gearbeitet wird, so dass die entstehenden Zersetzungsprodukte erst stromabwärts vom Fenster in merklicher Menge auftreten. Die Strömungsgeschwindigkeit wird dabei insbesondere nach der Art und dem Querschnitt des Reaktionsgefässes gewählt werden.

Als besonders vorteilhaft hat sich in solchen Fällen eine stufenweise Durchführung der thermischen Behandlung und der Lichteinwirkung erwiesen, wobei sich einzelne Stufen auch wiederholen können, z.B. in der Stufenfolge: a) Thermische Behandlung und/oder b) Lichteinwirkung

und/oder c) thermische Behandlung und/oder d) Lichteinwirkung. Die Lichteinwirkungsstufen können dabei abwechselnd mit kohärentem Licht und mit inkohärentem Licht erfolgen. Die Reaktanden werden also z.B. zuerst auf die Temperatur der thermischen Behandlung aufgeheizt und danach am Bestrahlungsfenster vorbeigeführt. Hierbei wird die Strömungsgeschwindigkeit zweckmässigerweise so gewählt, dass vor dem Passieren des Fensters bereits die für die photochemische Reaktion günstige Temperatur erreicht ist. Dabei kann nach der Bestrahlungsstufe nochmals eine thermische Behandlung erfolgen. Es kann aber weiter stromabwärts auch noch zusätzlich ein Laser eingebaut werden, wodurch sich z.B. die Stufenfolge a) thermische Behandlung, b) Bestrahlung mit einer Nieder- oder Mitteldrucklampe, c) thermische Behandlung und/oder d) Bestrahlung mit Laser ergibt. Ein Laser der Stufe d) kann bei Vorschaltung der Bestrahlungsstufe b) dabei mit wesentlich geringerer Energie als bei einer alleinigen Laserbestrahlungsstufe notwendig, betrieben werden, wodurch sich eine erhebliche Kosteneinsparung erreichen lässt.

Bei Verwendung einer Laserstrahlung hängt die optimale Wellenlänge von den spektroskopischen und photochemischen Eigenschaften der jeweiligen Reaktionspartner ab und kann zwischen 10 nm und 5000 nm liegen. Die Laserstrahlung kann gepulst oder kontinuierlich sein, d.h. zwischen $10^{-15}$ Sekunden oder $\infty$ Sekunden einwirken.

Zur weiteren Erläuterung der Erfindung dient die Zeichnung. In der beigefügten Zeichnung zeigt:

Fig. 1 eine Vorrichtung, bei der dem Ausgangsmaterial zunächst Energie in Form von Wärme zugeführt werden kann. Sie besteht aus einem Reaktor 1, der auf der einen Seite durch ein für das jeweilige Laserlicht durchlässiges Fenster 2 abgeschlossen ist. Ausserhalb dieses Fensters befindet sich dann die Strahlungsquelle 3, deren Strahlung durch das Fenster 2 in den Reaktionsraum 1 eintritt. Die umzusetzende Substanz wird über ein Ventil sowie entsprechende Rohrleitungen bei 4 in den Reaktionsraum eingeleitet. Die Reaktanden können sowohl durch den Reaktionsraum strömen als auch in ihm stehen. Ein Manometer dient nach dem Ventil zur Druckkontrolle. Nach Ablauf der chemischen Reaktion wird das Reaktionsgemisch bei 5 abgezogen und aufgearbeitet.

Eine spezielle Ausführungsform der obigen Vorrichtung für die Vinylchloridherstellung (VCM) zeigt Fig. 2 der Zeichnung. Sie besteht aus einem elektrisch aufheizbaren Quarzreaktor 11 von 15 × 2,5 cm. Der Reaktor ist an den Enden mit Quarzscheiben 12 versehen, durch die die Strahlung eines KrF Excimerenlasers 13 (Wellenlänge 249 nm; Impulsdauer $10^{-8}$ Sekunden, Impulsenergie 160 mJ) in den Reaktionsraum eintritt. Die Dichloräthanzufuhr erfolgt bei 14, der Abzug des Reaktionsgemisches zu einem Gaschromatographen bei 15. Die Reaktionstemperatur wird durch den Ofen 16 geregelt. Hinter dem Strahlungsaustrittsfenster ist ein Laserenergiemesser 17 angeordnet. Der Druck im Reaktor wird durch ein bei 18 angeschlossenes Manometer gemessen.

Fig. 3 der Zeichnung zeigt in graphischer Darstellung die Verbesserung der VCM-Ausbeute in Abhängigkeit von der Zahl der Laserpulse bei den im Beispiel 1 angegebenen Bedingungen und durch Kombination des thermischen mit dem photochemischen Verfahren unter den im Beispiel 2 angegebenen Bedingungen.

Als besonders vorteilhafte Ausführungsform des erfindungsgemässen Verfahrens bei der Anwendung auf die VCM-Herstellung wurde ein Zusatz von Sauerstoff oder Nitromethan, gegebenenfalls in Mischung mit Inertgas mit einem Partialdruck von 1 Pa bis 6,5 kPa gefunden. Hierdurch lässt sich eine sehr lange Kettenlänge erzielen, die pro Anregung $10^5$ bis $10^6$ Umsetzungen beträgt.

Verwendet man entgegen der erfindungsgemässen Lehre kohärentes Licht mit höherem Einfangquerschnitt, beispielsweise die der Anregungswellenlänge des Dichloräthans entsprechende Wellenlänge des Argonlasers mit 193 nm, so sind zwar infolge des höheren Energiegehalts weniger Lichtblitze erforderlich, anderseits kann dann aber auch das Vinylchlorid selbst schon absorbieren, so dass unerwünschte Polymerisationsreaktionen auftreten können. Arbeitet man hingegen unter den erfindungsgemässen Bedingungen, so beträgt die Reinheit des Reaktionsprodukts mehr als 99%. Auch gaschromatographisch lassen sich keine Verunreinigungen nachweisen.

Das Verfahren eignet sich sowohl für Reaktionen in der Gasphase als auch in der flüssigen Phase. Andere Beispiele für erfindungsgemässe Umsetzungen sind die Darstellung von

| | |
|---|---|
| Vinylfluorid | $C_2H_4FCl \rightarrow C_2H_3F + HCl$ |
| Tetrafluoräthylen | $C_2F_4HCl \rightarrow C_2F_4 + HCl$ |
| Chloropren | $C_4H_7Cl_2 \rightarrow C_4H_6Cl + HCl$ |
| Propylen | $C_3H_7Cl \rightarrow C_3H_6 + HCl$ |
| Vinylidenchlorid | $C_2H_3Cl_3 \rightarrow C_2H_2Cl_2 + HCl$. |

Gegenüber dem bekannten Verfahren ermöglicht daher die Erfindung die Durchführung der angestrebten Reaktion bei vergleichsweise niedrigerer Temperatur mit erheblich grösserer Geschwindigkeit unter Ausschaltung unerwünschter Nebenreaktionen. Da keine Festphasen-Katalysatoren erforderlich sind, besteht auch nicht die Gefahr einer Katalysatorvergiftung.

Gegenüber dem thermischen Verfahren lassen sich mit dem erfindungsgemässen Verfahren erhebliche Ausbeuteverbesserungen erreichen. So erhält man z.B. unter technischen Bedingungen durch Kombination mit einer einzigen Bestrahlungsstufe unter Verwendung einer Mitteldrucklampe Ausbeuteverbesserungen von bis zu 10%.

Im Vergleich zu anderen laserphotochemischen Verfahren weist das erfindungsgemässe Verfahren eine um Grössenordnung bessere Ausnutzung der Laserphotonen durch entsprechend der jeweiligen Reaktionskettenlänge vielfachen Umsetzung der primär durch die Laserstrahlung erzeugten aktiven Teilchen auf. Durch die erfindungsgemässe Kombination der Laserphotolyse mit einer Kettenreaktion wird daher auch die Wirtschaftlichkeit eines laserphotochemischen Verfahrens um ein Vielfaches gesteigert.

Als Reaktionsgefässe lassen sich auch die in den bekannten thermischen Verfahren technisch eingesetzten Reaktionsapparaturen nach zusätzlichem Einbau eines oder mehrerer strahlungsdurchlässiger Fenster verwenden.

Selbstverständlich sind weitere Varianten im Aufbau solcher Vorrichtungen möglich, z.B. durch Einbau weiterer lichtdurchlässiger Fenster für die Kombination mehrerer Strahlungsquellen.

Die folgenden Beispiele erläutern die Erfindung anhand der Herstellung von Vinylchloridmonomer (VCM).

Beispiel 1

Die Temperatur im Reaktor betrug 300 °C, der Druck etwa 70 kPa. Bei 20 Laserpulsen von $10^{-8}$ Sekunden Dauer und 160 mJ pro Puls wurden 50% Umsetzung zu VCM erzielt. Unter diesen Bedingungen und bei einer Absorptionsstrecke von 15 cm werden etwa 1% des Kryptonfluorid-Laserlichtes, d.h. etwa $2{,}1 \times 10^{15}$ KrF Laserphotonen absorbiert. Unter diesen Bedingungen werden jedoch $10^{19}$ Vinylchloridmoleküle pro Laserpuls gebildet, d.h. es werden mindestens 5000 Vinylchloridmoleküle pro absorbiertes Laserquant erzeugt, entsprechend einer Kettenlänge einer Radikalreaktion von $>5000$. Gaschromatographisch lassen sich keine Verunreinigungen nachweisen. Verunreinigungen müssen daher unter 1% liegen.

Der Energieaufwand zur Erzeugung der Laserphotonen beträgt nur etwa 5% der Energie, die erforderlich ist, um das Dichloräthan von 300 °C auf die 500 °C des bekannten technischen Verfahrens aufzuheizen.

Beispiel 2

Darstellung von monomerem Vinylchlorid (VCM) aus 1,2-Dichloräthan.

Es wurde mit der Vorrichtung der Fig. 2 gearbeitet. Als Strahlungsquelle diente a) ein Laser (Wellenlänge 308 nm, Impulsenergie 100 mJ) und b) eine Quecksilberdampfmitteldrucklampe (Wellenlänge 254 nm, Leistung 10 mW/$cm^2$). Die Reaktionstemperatur im Reaktor betrug 450 °C, der Druck 500 Torr.

Sowohl bei der Verwendung der Lichtquelle a) als auch von b) wurde eine wesentliche Steigerung des Umsetzungsgrades erreicht (vgl. Fig. 3).

**Patentansprüche**

1. Verfahren zur Herstellung von gegebenenfalls halogenierten Verbindungen mit wenigstens einer olefinischen Doppelbindung durch Abspaltung von Halogenwasserstoff aus der entsprechenden, Halogen und Wasserstoff enthaltenden gesättigten Verbindung, dadurch gekennzeichnet, dass man die gasförmige gesättigte Verbindung in einem Reaktionsraum mit gepulstem kohärentem Licht oder/und inkohärentem Licht bestrahlt und die Wellenlänge des eingestrahlten Lichts und die Druck- und Temperaturbedingungen im Reaktionsraum so wählt, dass sich ein Einfangquerschnitt von $10^{-15}$ bis $10^{-25}$ $cm^2$/Molekül ergibt.

2. Verfahren nach Anspruch 1, dadurch gekennzeichnet, dass man eine Strahlung einer Imulsdauer bis herab zu $10^{-15}$ Sekunden und einer Energie von 0,01 bis 100 Joule verwendet.

3. Verfahren nach Anspruch 1 oder 2, dadurch gekennzeichnet, dass man den Einfangquerschnitt durch Zusatz kleiner Mengen an höher halogenierten Verbindungen, Halogenwasserstoff oder Halogen regelt.

4. Verfahren nach einem der vorhergehenden Ansprüche, dadurch gekennzeichnet, dass man Dichloräthan einsetzt und Vinylchlorid herstellt.

5. Verfahren nach Anspruch 4, dadurch gekennzeichnet, dass man eine Temperatur zwischen 200 und 600 °C anwendet.

6. Verfahren nach Anspruch 5, dadurch gekennzeichnet, dass man bei Verwendung von kohärentem Licht eine Temperatur von 230 bis 320 °C anwendet.

7. Verfahren nach Anspruch 5, dadurch gekennzeichnet, dass man bei Verwendung von inkohärentem Licht eine Temperatur von 450 bis 550 °C anwendet.

8. Verfahren nach einem der Ansprüche 4 bis 7, dadurch gekennzeichnet, dass man einen Druck zwischen 50 und 3000 kPa anwendet.

9. Verfahren nach Anspruch 8, dadurch gekennzeichnet, dass man einen Druck zwischen 0,1 und 1 MPa anwendet.

10. Verfahren nach einem der Ansprüche 1 bis 5 und 7, dadurch gekennzeichnet, dass man als inkohärente Lichtquelle eine Metalldampfmitteldrucklampe oder -niederdrucklampe einsetzt.

11. Verfahren nach Anspruch 10, dadurch gekennzeichnet, dass man eine Quecksilber-, Thallium- und/oder Eisenmetalldampflampe verwendet.

12. Verfahren nach einem der Ansprüche 10 oder 11, dadurch gekennzeichnet, dass man mit Metalldampflampen mit einem Quantenflussbereich von 0,01 bis 10 W/$cm^2$ arbeitet.

13. Verfahren nach einem der Ansprüche 10 bis 12, dadurch gekennzeichnet, dass man mit einem Quantenflussbereich von 0,1 bis 2 W/$cm^2$ arbeitet.

14. Verfahren nach einem der Ansprüche 1 bis 13, dadurch gekennzeichnet, dass die Reaktanden den Reaktionsraum durchströmen.

15. Verfahren nach einem der Ansprüche 1 bis 14, dadurch gekennzeichnet, dass man die thermische Behandlung und die Lichteinwirkung stufenweise hintereinander durchführt, wobei sich einzelne Stufen auch wiederholen können.

16. Verfahren nach Anspruch 15, dadurch gekennzeichnet, dass man folgende Stufen hintereinanderschaltet:

a) thermische Behandlung und/oder
b) Lichteinwirkung und/oder
c) thermische Behandlung und/oder
d) Lichteinwirkung.

17. Verfahren nach einem der Ansprüche 15 oder 16, dadurch gekennzeichnet, dass hintereinander erfolgende Lichteinwirkungsstufen abwech-

selnd mit kohärentem Licht und mit inkohärentem Licht arbeiten.

18. Verfahren nach einem der Ansprüche 4 bis 17, dadurch gekennzeichnet, dass man mit Dichloräthan Sauerstoff oder Nitromethan, gegebenenfalls in Mischung mit Inertgas, einem Partialdruck zwischen 1 Pa bis 6,5 kPa zusetzt.

**Claims**

1. Process for the preparation of optionally halogenated compounds with at least one olefinic double bond by the splitting off of hydrogen halide from the corresponding halogen- and hydrogencontaining saturated compound, characterised in that one irradiates the gaseous saturated compound in a reaction chamber with pulsed coherent light and/or incoherent light and so selects the wavelength of the irradiated light and the pressure and temperature conditions in the reaction chamber that a capture cross-section of $10^{-15}$ to $10^{-25}$ cm²/molecule results.

2. Process according to claim 1, characterised in that one uses a radiation with an impulse period of down to $10^{-15}$ seconds and an energy of 0.01 to 100 Joule.

3. Process according to claim 1 or 2, characterised in that one regulates the capture cross-section by the addition of small amounts of more highly halogenated compounds, hydrogen halide or halogen.

4. Process according to one of the preceding claims, characterised in that one uses dichloroethane and produces vinyl chloride.

5. Process according to claim 4, characterised in that one uses a temperature between 200 and 600 °C.

6. Process according to claim 5, characterised in that, in the case of the use of coherent light, one uses a temperature of 230 to 320 °C.

7. Process according to claim 5, characterised in that, in the case of the use of incoherent light, one uses a temperature of 450 to 550 °C.

8. Process according to one of claims 4 to 7, characterised in that one uses a pressure between 50 and 3000 kPa.

9. Process according to claim 8, characterised in that one uses a pressure between 0.1 and 1 MPa.

10. Process according to one of claims 1 to 5 and 7, characterised in that, as source of incoherent light, one uses a metal vapour medium pressure lamp or low pressure lamp.

11. Process according to claim 10, characterised in that one uses a mercury, thallium and/or iron metal vapour lamp.

12. Process according to one of claims 10 or 11, characterised in that one operates with metal vapour lamps with a quantum flux range of 0.01 to 10 W/cm².

13. Process according to one of claims 10 to 12, characterised in that one operates with a quantum flux range of 0.1 to 2 W/cm².

14. Process according to one of claims 1 to 13, characterised in that the reactants flow through the reaction chamber.

15. Process according to one of claims 1 to 14, characterised in that one carries out the thermal treatment and the action of light consecutively stepwise, whereby individual steps can also be repeated.

16. Process according to claim 15, characterised in that one consecutively carries out the following steps:
a) thermal treating and/or
b) action of light and/or
c) thermal treating and/or
d) action of light.

17. Process according to one of claims 15 or 16, characterised in that successively following light action steps operate alternatingly with coherent light and with incoherent light.

18. Process according to one of claims 4 to 17, characterised in that with dichloroethane, one adds oxygen or nitromethane, optionally in admixture with inert gas, with a partial pressure between 1 Pa and 6.5 kPa.

**Revendications**

1. Procédé de préparation de composés éventuellement halogénés et contenant au moins une double liaison oléfinique par élimination d'un hydracide halogéné à partir du composé saturé correspondant contenant un halogène et de l'hydrogène, caractérisé en ce que l'on irradie le composé saturé gazeux dans une chambre de réaction par de la lumière cohérente pulsée et/ou de la lumière incohérente et on choisit la longueur d'onde de la lumière d'irradiation et les conditions de pression et de température dans la chambre de réaction de manière que la section efficace d'absorption soit de $10^{-15}$ à $10^{-25}$ cm²/molécule.

2. Procédé selon la revendication 1, caractérisé en ce qu'on utilise une radiation d'une durée d'impulsion pouvant descendre jusqu'à $10^{-15}$ seconde et d'une énergie de 0,01 à 100 joules.

3. Procédé selon la revendication 1 ou 2, caractérisé en ce qu'on règle la section efficace d'absorption par addition de petites quantités de composés plus fortement halogénés, d'un halogénure d'hydrogène ou d'un halogène.

4. Procédé selon l'une des revendications qui précèdent, caractérisé en ce qu'on met en œuvre du dichloréthane et on prépare du chlorure de vinyle.

5. Procédé selon la revendication 4, caractérisé en ce qu'on utilise une température de 200 à 600 °C.

6. Procédé selon la revendication 5, caractérisé en ce que, lorsqu'on utilise une lumière cohérente, on utilise une température de 230 à 320 °C.

7. Procédé selon la revendication 5, caractérisé en ce que, lorsqu'on utilise une lumière incohérente, on utilise une température de 450 à 550 °C.

8. Procédé selon l'une des revendications 4 à 7, caractérisé en ce que l'on opère à une pression de 50 à 3000 kPa.

9. Procédé selon la revendication 8, caractérisé en ce que l'on opère à une pression de 0,1 à 1 MPa.

10. Procédé selon l'une des revendications 1 à 5 et 7, caractérisé en ce que l'on utilise, en tant que source de lumière incohérente, une lampe à vapeurs métalliques à pression moyenne ou à basse pression.

11. Procédé selon la revendication 10, caractérisé en ce que l'on utilise une lampe à vapeurs de mercure, de thallium et/ou de fer métallique.

12. Procédé selon l'une des revendications 10 ou 11, caractérisé en ce que l'on opère avec des lampes à vapeurs métalliques dans un domaine de flux de quanta de 0,01 à 10 $W/cm^2$.

13. Procédé selon les revendications 10 à 12, caractérisé en ce que l'on opère avec un domaine de flux de quanta de 0,1 à 2 $W/cm^2$.

14. Procédé selon l'une des revendications 1 à 13, caractérisé en ce que les réactifs s'écoulent au travers de la chambre de réaction.

15. Procédé selon l'une des revendications 1 à 14, caractérisé en ce que l'on réalise le traitement thermique et l'action de la lumière en stades successifs, et en répétant éventuellement des stades individuels.

16. Procédé selon la revendication 15, caractérisé en ce que l'on réalise successivement les stades opératoires suivants:

a) traitement thermique et/ou
b) action de la lumière et/ou
c) traitement thermique et/ou
d) action de la lumière.

17. Procédé selon l'une des revendications 15 ou 16, caractérisé en ce que, dans les stades successifs d'action de la lumière, on opère alternativement avec une lumière cohérente et une lumière incohérente.

18. Procédé selon l'une des revendications 4 à 17, caractérisé en ce que l'on ajoute au dichloréthane de l'oxygène ou du nitro-méthane, éventuellement en mélange avec un gaz inerte, à une pression partielle de 1 Pa à 6,5 kPa.

FIG.1

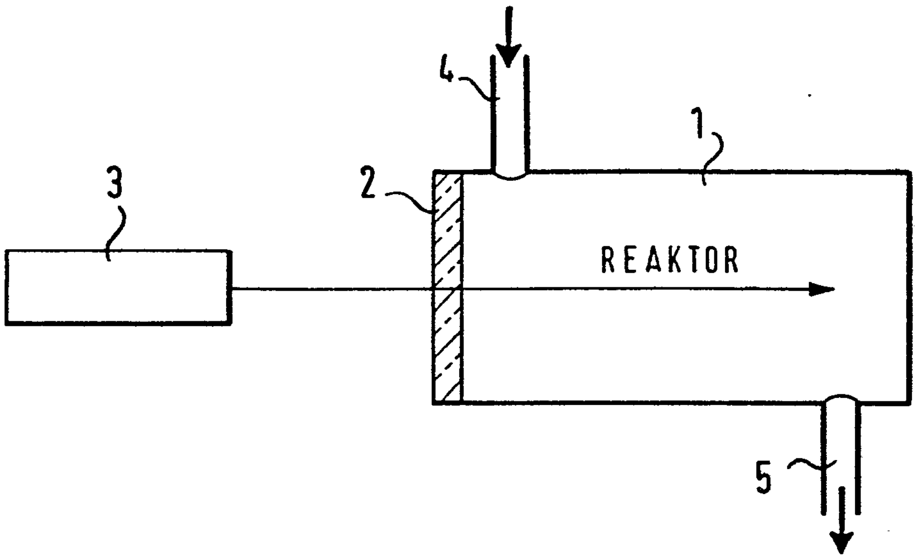


FIG.2

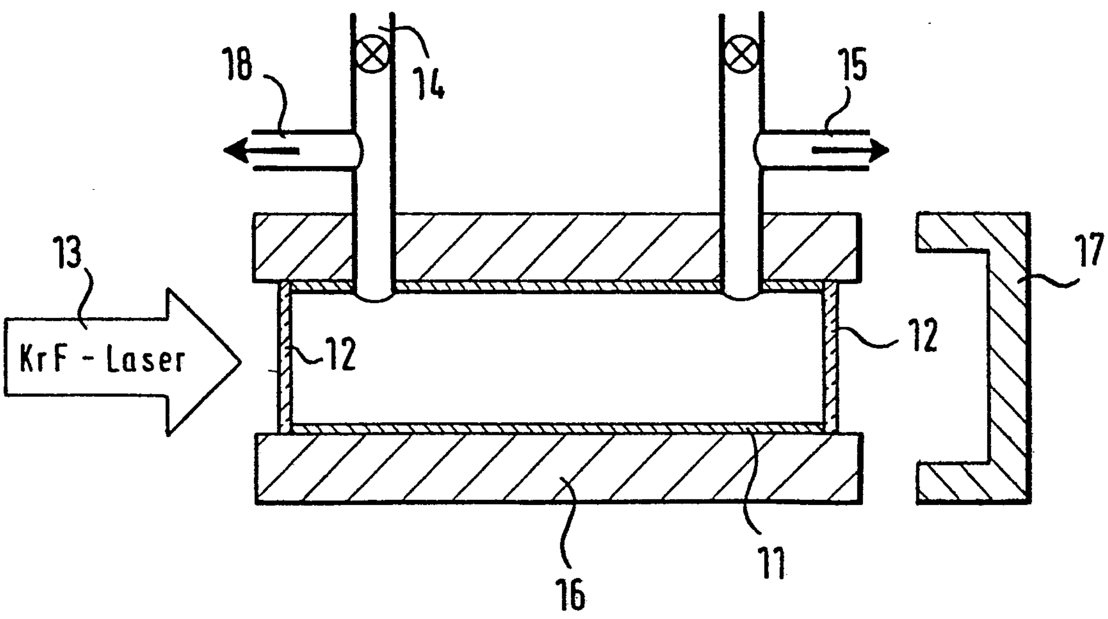

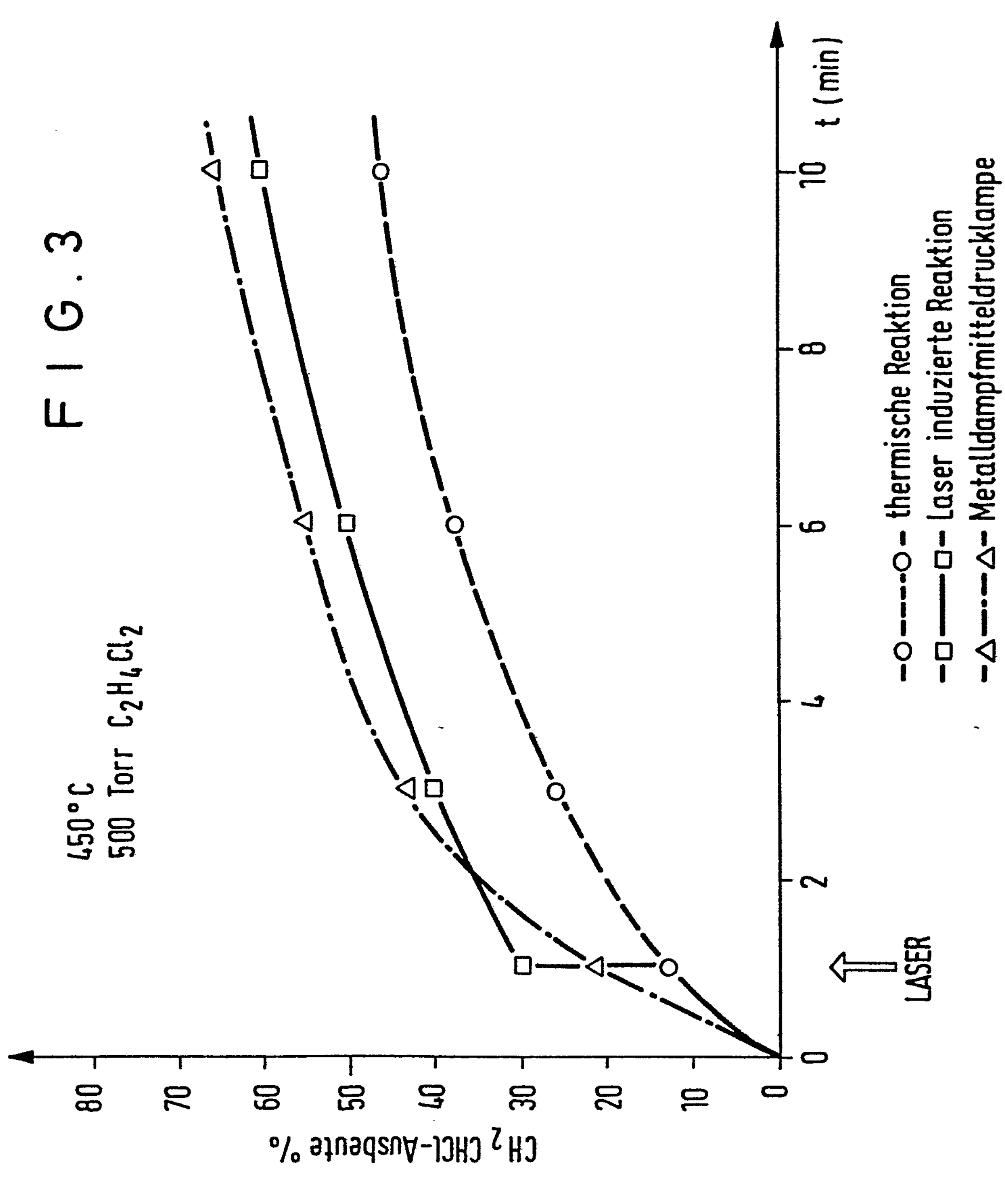
450°C
500 Torr $C_2H_4Cl_2$
$CH_2$ CHCl-Ausbeute %
0
10
20
30
40
50
60
70
80
0
2
4
6
8
10
t (min)
LASER
thermische Reaktion
Laser induzierte Reaktion
Metalldampfmitteldrucklampe

FIG. 3